# FASCICULE DE BREVET EUROPEEN

(11) **EP 1 604 703 B1**
(45) Date de publication et mention de la délivrance du brevet: **24.08.2011**
(21) Numéro de dépôt: 05300461.0
(22) Date de dépôt: 07.06.2005
(51) Int. Cl.: A61M 27/00

(54) **Valve sous-cutanée**
Subkutanventil
Valve for subcutaneous use

(30) Priorité: 11.06.2004 FR 0451264
(43) Date de publication de la demande: 14.12.2005
(73) Titulaire: SOPHYSA, F-91893 Orsay Cedex (FR)
(72) Inventeur: Cabaud, François, 25870 Chatillon-le-Duc (FR); Coneau, Pascal, 70150 Etuz (FR); Negre, Philippe, 75116 Paris (FR)
(74) Mandataire: Leszczynski, André

(56) Documents cités:
- EP-A- 0 688 575
- EP-A- 1 205 210
- FR-A- 2 804 331
- US-A- 4 673 384
- US-A- 5 637 083

## Description

La présente invention a pour objet une valve sous-cutanée destinée à des applications thérapeutiques, la valve étant contrôlable depuis l'extérieur pour modifier à travers des tissus cutanés un passage ou une distribution de liquide dans des prothèses ou des systèmes implantés.

Parmi les applications thérapeutiques, on peut citer le traitement de l'hydrocéphalie qui consiste à dériver le liquide céphalorachidien contenu dans les ventricules de la cavité crânienne vers tout autre site de résorption.

On connaît par le brevet européen EP-B1-688 575 de la déposante une telle valve, comportant un rotor pourvu d'un ressort à lame courbe fixé dessus, le ressort à lame venant s'appliquer contre une bille pour la maintenir contre un orifice d'entrée de la valve de manière à réguler le passage de liquide par cet orifice d'entrée. La rotation du rotor d'une position angulaire d'indexation à une autre entraîne le glissement du point de contact de la bille sur le ressort à lame et donc une modification de la contrainte exercée par le ressort à lame sur la bille. Le rotor peut être verrouillé/déverrouillé par attraction et/ou répulsion mutuelle de micro-aimants disposés sur le rotor.

On connaît par le brevet US 4 673 384 de la déposante une autre valve sous-cutanée comportant également un rotor pourvu d'un ressort à lame courbe fixé dessus au moyen d'un élément intermédiaire. La valve comporte également des éléments d'arrêt permettant de définir deux configurations de la valve en fonction de la position debout ou couchée d'un patient.

L'invention vise à améliorer une valve sous-cutanée du type précité.

L'invention a ainsi pour objet une valve sous-cutanée comportant :
- un corps définissant une chambre et comportant un orifice d'entrée et un orifice de sortie débouchant dans la chambre,
- un obturateur apte à obturer au moins partiellement l'orifice d'entrée,
- un organe de rappel élastique agencé pour maintenir l'obturateur contre l'orifice d'entrée afin de réguler le passage de liquide par cet orifice d'entrée,
- un rotor logé dans la chambre,
la valve étant caractérisée par le fait que le rotor comporte une surface formant une came et par le fait que l'organe de rappel élastique vient en appui sur ladite surface du rotor en formant un contact mobile avec ladite surface.

Grâce à l'invention, l'organe de rappel élastique peut être indépendant du rotor, c'est-à-dire ne pas être fixé à celui-ci. Le rotor peut présenter ainsi une structure relativement simple, et notamment comporter un nombre réduit de pièces constitutives.

Par ailleurs, lorsque l'organe de rappel élastique comporte un ressort à lame, la longueur de celui-ci peut être réduite, s'étendant par exemple sur un secteur angulaire inférieur à 180°, par exemple sensiblement égal à 90°. En effet, dans l'invention, le ressort à lame n'est pas fixé sur le rotor et l'obturateur n'a pas à glisser sur le ressort à lame.

En outre, l'invention permet un réglage relativement précis de la contrainte exercée par l'organe de rappel élastique sur l'obturateur grâce à la présence de la surface formant came.

Avantageusement, l'organe de rappel élastique est agencé pour permettre un contact permanent de celui-ci sur la surface formant came du rotor.

Dans un exemple de mise en oeuvre de l'invention, l'organe de rappel élastique est fixé, notamment à une extrémité, sur le corps de la valve.

L'organe de rappel élastique peut comporter un ressort à lame, notamment un ressort à lame courbe, avec notamment une extrémité libre venant en appui sur l'obturateur avec une contrainte prédéterminée.

Le ressort à lame comporte avantageusement une portion en saillie venant en appui sur la surface formant came.

En variante, l'organe de rappel élastique comporte un élément d'appui fixé sur le ressort à lame et apte à glisser sur la surface formant came.

Grâce à la portion en saillie du ressort à lame ou de l'élément d'appui précités, une rotation du rotor entraîne une déflexion du ressort à lame, ce qui permet de modifier la contrainte exercée sur l'obturateur par le ressort à lame.

La forme de la surface formant came est choisie en fonction d'une gamme souhaitée de contraintes à exercer sur l'obturateur.

Le ressort à lame peut avantageusement exercer une contrainte sur la portion en saillie ou sur l'élément d'appui précités, de manière à assurer un contact permanent de ladite portion en saillie ou de l'élément d'appui sur la surface formant came.

L'organe de rappel élastique peut comporter un unique ressort à lame. En variante, l'organe de rappel élastique peut comporter un ressort à lame additionnel, agencé notamment pour accroître la contrainte exercée sur la portion en saillie du ressort à lame ou l'élément d'appui sur la surface formant came. L'organe de rappel élastique peut par exemple comporter deux ressorts à lame assemblés.

Dans un autre exemple de mise en oeuvre de l'invention, l'organe de rappel élastique est indépendant du corps de la valve, c'est-à-dire n'est pas fixé sur le corps.

L'organe de rappel élastique peut comporter un ressort, notamment hélicoïdal, lequel est notamment solidaire à une extrémité d'un élément d'appui apte à glisser sur la surface formant came, l'autre extrémité du ressort étant notamment en appui sur l'obturateur.

Dans un exemple de mise en oeuvre de l'invention, un ressort additionnel est disposé entre, d'une part, l'élément d'appui et, d'autre part, le corps de la valve ou un insert fixé sur ce corps.

Le ressort en appui sur l'obturateur peut être disposé de manière à être entouré par le ressort additionnel par exemple.

L'élément d'appui peut être constitué par une portion d'une cage pourvue d'un ou de plusieurs orifices ménageant un passage entre l'orifice d'entrée précité et l'intérieur de la valve.

Avantageusement, la surface formant came s'étend sur tout le pourtour du rotor.

Ainsi l'invention permet d'atteindre une gamme de contraintes ou pressions relativement large car la gamme de positions angulaires d'indexation du rotor peut s'étendre de 0 à 360°.

Dans un exemple de mise en oeuvre de l'invention, la surface formant came présente un rayon croissant sur un secteur angulaire supérieur à 180°, notamment sensiblement égal à 360°.

La surface formant came peut être sensiblement continue ou comporter au moins un renfoncement ou encoche, notamment une pluralité de renfoncements ou encoches répartis autour de l'axe de rotation du rotor, dans lequel ou lesquels peut s'engager une portion de l'organe de rappel élastique. Ces renfoncements correspondent avantageusement à des positions angulaires d'indexation du rotor.

De préférence, l'obturateur comporte une bille.

De préférence encore, le rotor comporte deux micro-aimants mobiles linéairement par rapport au rotor suivant une direction sensiblement radiale et aptes à coopérer avec des moyens de verrouillage du rotor dans une position angulaire prédéterminée.Dans un exemple de mise en oeuvre de l'invention, la hauteur du centre de gravité du rotor, mesurée suivant un axe perpendiculaire au rotor, est constante lorsque le rotor est déplacé en rotation.

L'invention pourra être mieux comprise à la lecture de la description détaillée qui va suivre, d'exemples de mise en oeuvre non limitatifs de l'invention, et à l'examen du dessin annexé, sur lequel :
- la figure 1 représente, schématiquement et partiellement, en coupe axiale, une valve sous-cutanée conforme à l'invention,
- la figure 2 est une vue schématique et partielle de deux micro-aimants destinés à équiper la valve de la figure 1,
- la figure 3 représente, schématiquement et partiellement, en coupe axiale, le rotor de la valve de la figure 1,
- la figure 4 représente, schématiquement et partiellement, en coupe axiale, une valve selon un autre exemple de mise en oeuvre de l'invention, et
- les figures 5 à 9 illustrent, schématiquement et partiellement, d'autres variantes de mise en oeuvre de l'invention.

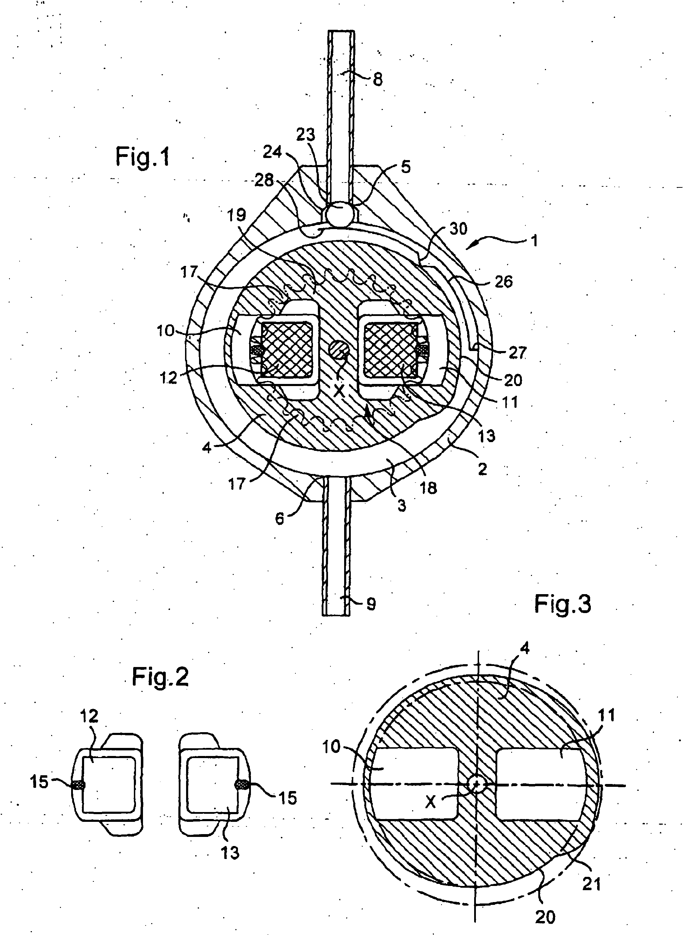

On a représenté sur la figure 1 une valve sous-cutanée 1 conforme à l'invention, comportant un corps 2 définissant une chambre 3 sensiblement cylindrique d'axe X dans laquelle est logé un rotor 4.

Le corps 2 comporte des orifices d'entrée 5 et de sortie 6 débouchant dans la chambre 3.

Un conduit d'entrée 8 et un conduit d'évacuation 9 sont fixés sur le corps 2 et débouchent respectivement sur les orifices d'entrée 5 et de sortie 6.

Le conduit d'entrée 8 et le conduit d'évacuation 9 peuvent être connectés respectivement à un cathéter d'amenée et un cathéter de drainage de liquide, non représentés sur le dessin.

Le rotor 4 comporte deux logements 10 et 11 aptes à recevoir chacun un micro-aimant 12 et 13.

Chaque micro-aimant 12, 13 est agencé de manière à pouvoir coulisser linéairement dans le logement 10, 11 correspondant suivant une direction sensiblement radiale. Ces micro-aimants 12, 13 comportent chacun un relief de verrouillage 15, comme illustré sur la figure 2.

Ces reliefs 15 peuvent comporter par exemple chacun un ergot cylindrique.

Ces reliefs 15 sont aptes à s'engager dans des encoches 17 de moyens de verrouillage 18 de la valve 1.

Dans l'exemple considéré, ces moyens de verrouillage 18 comportent une partie centrale 19 fixe par rapport au corps 2 et sur la périphérie de laquelle sont réalisées les encoches 17. Ces dernières sont régulièrement réparties tout autour de l'axe X.

Grâce à un dispositif de réglage externe, non représenté sur les figures, il est possible de déplacer les micro-aimants 12 et 13 simultanément dans leurs logements respectifs 10 et 11, radialement vers l'extérieur, afin de désengager les reliefs 15 des encoches 17.

Ce désengagement permet de faire tourner le rotor 4 autour de l'axe X d'une position angulaire d'indexation vers une autre.

Le dispositif de réglage externe permet également de repositionner les micro-aimants 12 et 13 dans une position verrouillée dans laquelle les reliefs 15 sont engagés dans des encoches 17.

Il est possible de se référer au brevet EP-B1-688 575 pour plus de détails quant à la structure du dispositif de réglage externe.

Le rotor 4 comporte sur tout son pourtour une surface 20 formant une came.

Comme on peut le voir sur la figure 3 notamment, cette surface 20 présente un rayon croissant sur un secteur angulaire sensiblement égal à 360°, dans le sens des aiguilles d'une montre, et comporte une zone 21 de faible longueur présentant une pente décroissante relativement forte.

La valve 1 comporte un obturateur 23 constitué d'une bille, laquelle est maintenue contre un siège tronconique 24 en regard de l'orifice d'entrée 5.

L'obturateur 23 est maintenu contre cet orifice d'entrée 5 au moyen d'un organe de rappel élastique 26.

Dans l'exemple considéré, l'organe de rappel élastique 26 est un ressort à lame courbe fixé à une extrémité 27 sur une paroi latérale du corps de valve 2 et venant en appui à une extrémité libre 28 contre l'obturateur 23.

Le ressort à lame 26 comporte en outre une portion 30 en saillie venant au contact de la surface formant came 20 du rotor 4, comme on peut le voir sur la figure 1. Cette portion 30 peut être réalisée par exemple par pliage du ressort à lame.

Le ressort à lame 26 est agencé pour s'appliquer via la portion en saillie 30 sur la surface formant came 20 avec une certaine contrainte.

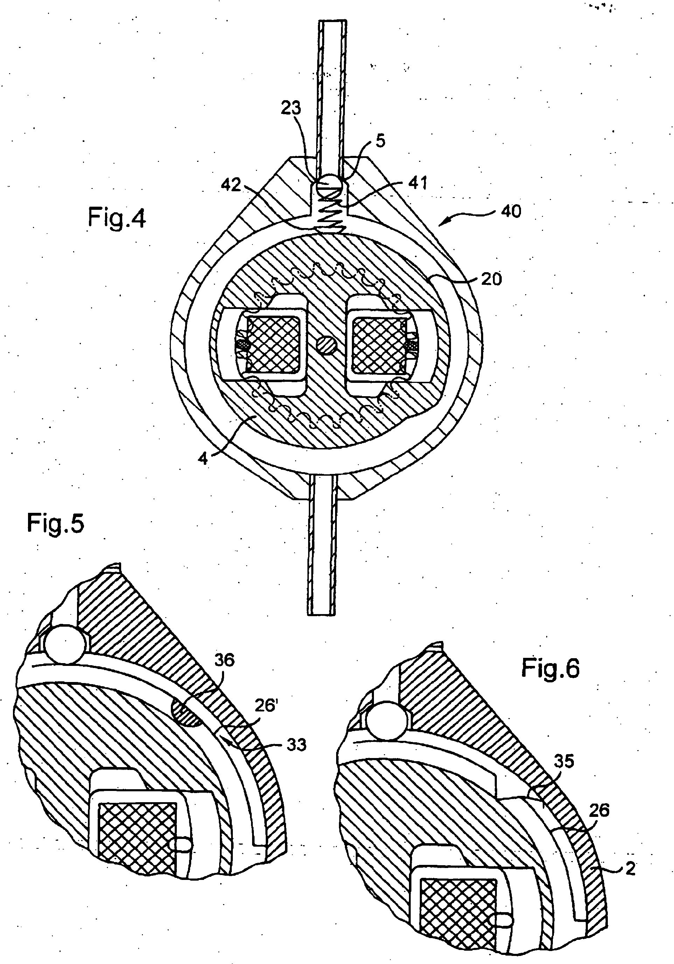

Pour accroître cette contrainte du ressort à lame 26 sur la surface 20, il est possible, comme illustré sur la figure 6, de prévoir un ressort à lame 35 additionnel qui vient en appui sur la paroi latérale de la chambre 3.

Lorsque la position angulaire du rotor 4 est modifiée, la surface formant came 20 provoque une déflexion du ressort à lame 26 et modifie par conséquent la contrainte exercée par ce ressort à lame 26 sur l'obturateur 23.

Dans les exemples qui viennent d'être décrits, le contact entre l'organe de rappel élastique 26 et la surface formant came 20 est réalisé par une portion du ressort à lame 26 lui-même.

En variante, comme illustré sur la figure 5, l'organe de rappel élastique 33 comporte un élément d'appui 36 fixé sur le ressort à lame 26'.

Cet élément d'appui 36 peut par exemple être réalisé en matière plastique, en métal ou en un matériau dur, et être assemblé avec le ressort à lame 26'.

On a représenté sur la figure 4 une valve 40 conforme à un autre exemple de mise en oeuvre de l'invention. Cette valve 40 se distingue de la valve 1 précédemment décrite par le fait que l'obturateur 23 est maintenu contre l'orifice d'entrée 5 non pas à l'aide d'un ressort à lame, mais à l'aide d'un ressort hélicoïdal 41 dont une extrémité vient en appui contre l'obturateur 23 et l'autre extrémité est solidaire d'un élément d'appui 42 apte à glisser sur la surface formant came 20 du rotor 4. Cet élément d'appui 42 peut être réalisé en matière plastique, en métal ou en un matériau dur.

En modifiant la position angulaire du rotor 4, la surface 20 exerce une contrainte variable sur le ressort hélicoïdal 41 de manière à modifier la pression exercée sur l'obturateur 23.

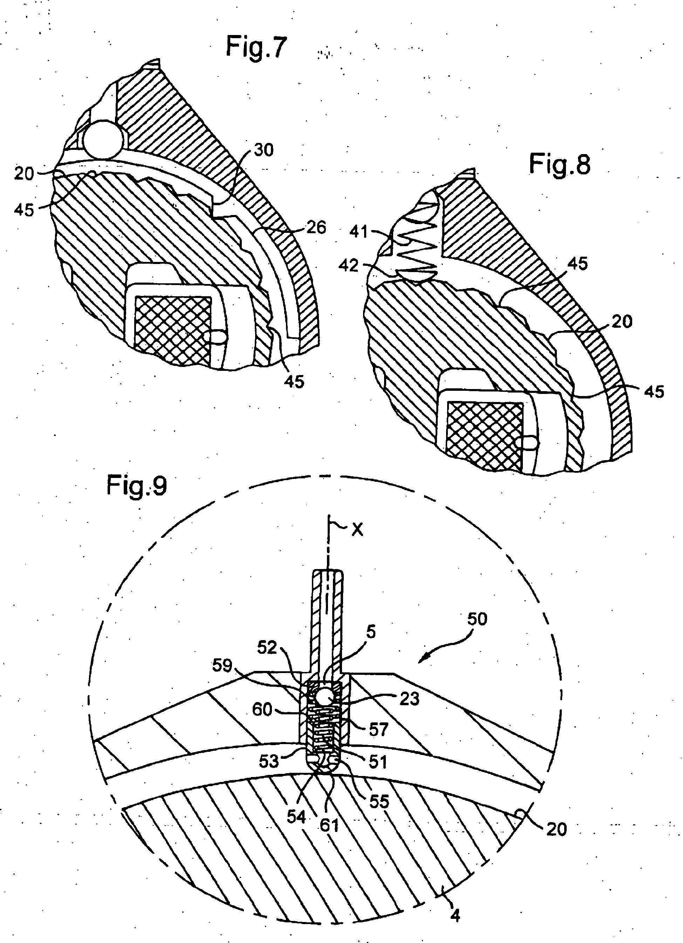

On a représenté sur la figure 9 une valve sous-cutanée 50 conforme à un autre exemple de mise en oeuvre de l'invention.

La valve 50 comporte, à l'instar de l'exemple décrit en référence à la figure 4, un obturateur 23 maintenu contre l'orifice d'entrée 5 en appui sur un siège 52, par un premier ressort 51, notamment hélicoïdal.

Le premier ressort 51 est en appui à son extrémité inférieure contre un épaulement 54 d'une cage 53.

Cette cage 53 comporte une pluralité d'orifices 55 ménageant un passage d'écoulement entre l'orifice d'entrée 5 et l'intérieur de la valve 50.

La cage 53 est montée de manière coulissante selon un axe X dans un logement 57.

La cage 53 est repoussée contre la surface formant came 20 du rotor 4 par l'intermédiaire d'un deuxième ressort 59, notamment hélicoïdal, disposé entre le fond du logement 57 et l'extrémité supérieure 60 de la cage 53.

Le deuxième ressort 59 présente un diamètre intérieur suffisant pour pouvoir recevoir le premier ressort 51.

La cage 53 comporte une portion inférieure 61, sensiblement hémisphérique par exemple, formant un élément d'appui venant au contact de la surface formant came 20 du ressort 4.

La disposition des deux ressorts 51 et 59 permet d'accroître la contrainte exercée par l'élément d'appui 61 sur la surface formant came 20.

Dans les exemples qui viennent d'être décrits, la surface formant came 20 est sensiblement continue, c'est-à-dire dépourvue d'encoches.

On ne sort pas du cadre de la présente invention lorsque la surface formant came 20 présente une pluralité d'encoches 45 réparties par exemple régulièrement autour de l'axe X, comme illustré sur les figures 7 et 8.

Ces encoches 45 peuvent être configurées de manière à correspondre à des positions angulaires d'indexation du rotor 4 dans le corps 2.

Comme illustré sur la figure 7, lorsque l'organe de rappel élastique est constitué par un ressort à lame 26, celui-ci peut comporter une portion en saillie 30 pouvant s'engager dans des encoches 45.

Comme illustré sur la figure 8, lorsque l'organe de rappel élastique comporte un élément d'appui 42 fixé sur un ressort hélicoïdal 41, l'élément d'appui 42 peut être agencé pour s'engager dans une encoche 45 du rotor 4.

Bien entendu, l'invention n'est pas limitée aux exemples de mise en oeuvre qui viennent d'être décrits.

On peut prévoir notamment une surface formant came qui s'étend seulement sur un secteur angulaire inférieur à 360°, et par exemple sur un secteur angulaire d'environ 180°.

## Revendications

1. Valve sous-cutanée (1 ; 40) comportant :
- un corps (2) définissant une chambre (3) et comportant un orifice d'entrée (5) et un orifice de sortie (6) débouchant dans la chambre (3),
- un obturateur (23) apte à obturer au moins partiellement l'orifice d'entrée (5),
- un organe de rappel élastique (26 ; 41, 42 ; 51) agencé pour maintenir l'obturateur (23) contre l'orifice d'entrée afin de réguler le passage de liquide par cet orifice d'entrée,
- un rotor (4) logé dans la chambre,
la valve étant **caractérisée par le fait que** le rotor (4) comporte une surface formant came (20) et **par le fait que** l'organe de rappel élastique (26 ; 41, 42 ; 51) vient en appui sur ladite surface du rotor en formant un contact mobile avec ladite surface.

2. Valve selon la revendication 1, **caractérisée par le fait que** l'organe de rappel élastique (26) est fixé, notamment à une extrémité, sur le corps de la valve.

3. Valve selon l'une des revendications précédentes, **caractérisée par le fait que** l'organe de rappel élastique comporte un ressort à lame courbe (26).

4. Valve selon la revendication 3. **caractérisée par le fait que** l'organe de rappel élastique (26) est un ressort à lame courbe fixé à une extrémité (27) sur une paroi latérale du corps de valve (2) et venant en appui à une extrémité libre (28) contre l'obturateur (23).

5. Valve selon la revendication 3 ou 4, **caractérisée par le fait que** le ressort à lame comporte une portion en saillie (30) venant en appui sur la surface formant came (20).

6. Valve selon la revendication 5, **caractérisée par le fait que** la portion en saillie (30) est réalisée par pliage du ressort à lame (26).

7. Valve selon la revendication 3 ou 4, **caractérisée par le fait que** l'organe de rappel élastique comporte un élément d'appui (36) fixé sur le ressort à lame (26') et apte à glisser sur la surface formant came.

8. Valve selon l'une quelconque des revendications 3 à 74, **caractérisée par le fait que** l'organe de rappel élastique comporte un unique ressort à lame (26).

9. Valve selon l'une quelconque des revendications 3 à 74, **caractérisée par le fait que** l'organe de rappel élastique comporte au moins deux ressorts à lame (26 ; 35) assemblés.

10. Valve selon la revendication 1, **caractérisée par le fait que** l'organe de rappel élastique (41) est indépendant du corps de la valve.

11. Valve selon la revendication 10, **caractérisée par le fait que** l'organe de rappel élastique comporte un ressort, notamment hélicoïdal (41 ; 51), lequel est notamment solidaire à une extrémité d'un élément d'appui (42) apte à glisser sur la surface formant came, l'autre extrémité du ressort étant notamment en appui sur l'obturateur.

12. Valve selon la revendication 11, **caractérisée par le fait qu'**un ressort additionnel (59) est disposé entre, d'une part, l'élément d'appui (53) et, d'autre part, le corps de la valve ou un insert fixé sur ce corps.

13. Valve selon l'une quelconque des revendications précédentes, **caractérisée par le fait que** la surface formant came s'étend sur le pourtour du rotor.

14. Valve selon la revendication 13, **caractérisée par le fait que** la surface formant came s'étend sur tout le pourtour du rotor.

15. Valve selon l'une quelconque des revendications précédentes, **caractérisée par le fait que** la surface formant came présente un rayon croissant sur un secteur angulaire supérieur à 180°.

16. Valve selon la revendication 15, **caractérisée par le fait que** la surface formant came présente un rayon croissant sur un secteur angulaire sensiblement égal à 360°.

17. Valve selon l'une quelconque des revendications précédentes, **caractérisée par le fait que** la surface formant came comporte au moins un renfoncement (45), notamment une pluralité de renfoncements répartis tout autour de l'axe de rotation du rotor, dans lequel ou lesquels peut s'engager une portion de l'organe de rappel élastique.

18. Valve selon l'une quelconque des revendications précédentes, **caractérisée par le fait que** l'obturateur comporte une bille (23).

19. Valve selon l'une quelconque des revendications précédentes, **caractérisée par le fait que** le rotor comporte deux micro-aimants (12 ; 13) mobiles linéairement par rapport au rotor (40) suivant une direction sensiblement radiale et aptes à coopérer avec des moyens de verrouillage (18) du rotor dans une position angulaire prédéterminée.

## Claims

1. A subcutaneous valve (1; 40) comprising:
· a body (2) defining a chamber (3) and comprising an inlet orifice (5) and an outlet orifice (6) opening out into the chamber (3);
· a shutter member (23) suitable for closing the inlet orifice (5), at least in part;
· a resilient return member (26; 41; 42; 51) configured to hold the shutter member (23) against the inlet orifice so as to regulate the passage of liquid through said inlet orifice; and
· a rotor (4) housed in the chamber;
the valve being **characterized in that** the rotor (4) has a cam-forming surface (20), and **in that** the resilient return member (26; 41; 42; 51) bears against said surface of the rotor by forming a moving contact with said surface.

2. A valve according to claim 1, **characterized in that** the resilient return member (26) is secured, in particular via an end, to the valve body.

3. A valve according to any one of the preceding claims, **characterized in that** the resilient return member comprises a curved spring blade (26).

4. A valve according to claim 3, **characterized in that** the resilient return member (26) is a curved spring blade fixed at one end (27) on a lateral wall of the valve body (2) and bearing with a free end (28) against the shutter member (23).

5. A valve according to claim 3 or 4, **characterized in that** the spring blade comprises a projecting portion (30) which comes to bear against the cam-forming surface (20).

6. A valve according to claim 5, **characterized in that** the projecting portion (30) results from a folding of the spring blade (26).

7. A valve according to claim 3 or 4, **characterized in that** the resilient return member comprises a first element (36) fastened to the spring blade (26') and suitable for sliding on the cam-forming surface.

8. A valve according to any one of claims 3 to 7, **characterized in that** the resilient return member comprises a single spring blade (26).

9. A valve according to any one of claims 3 to 7, **characterized in that** the resilient return member comprises at least two assembled-together spring blades 26; 35).

10. A valve according to claim 1, **characterized in that** the resilient return member (41) is independent of the valve body.

11. A valve according to claim 10, **characterized in that** the resilient return member comprises a spring, in particular a helical spring (41; 51), which is, in particular, secured at one end to a bearing element (42) suitable for sliding on the cam-forming surface, with the other end of the spring bearing in particular against the shutter member.

12. A valve according to claim 11, **characterized in that** an additional spring (59) is disposed between the bearing element (53) in one hand, and the valve body or an insert fastened to the body on the other hand.

13. A valve according to any one of the preceding claims, **characterized in that** the cam-forming surface extends around the periphery of the rotor.

14. A valve according to claim 13, **characterized in that** the cam-forming surface extends around the entire periphery of the rotor

15. A valve according to claim any one of the preceding claims, 1 **characterized in that** the cam-forming surface presents a radius that increases over an angular sector of more than 180°.

16. A valve according to claim 15, **characterized in that** the cam-forming surface presents a radius that increases over an angular sector substantially equal to 360°,

17. A valve according to any one of the preceding claims, **characterized in that** the cam-forming surface includes at least one setback (45), in particular a plurality of setbacks distributed all around the axis of rotation of the rotor, in which a portion of the resilient return member can be engaged.

18. A valve according to any one of the preceding claims, **characterized in that** the shutter member comprises a ball (23).

19. A valve according to any one of the preceding claims, **characterized in that** the rotor includes two micromagnets (12; 13) that are movable linearly relative to the rotor (40) in a direction that is substantially radial, and that are suitable for co-operating with means (18) for locking the rotor in a predetermined angular position.

## Patentansprüche

1. Subkutanventil (1; 40) mit:
- einem Körper (2), der eine Kammer (3) bildet und eine Einlassöffnung (5) und eine Auslassöfnung (6) aufweist, die in die Kammer (3) münden,
- einem Verschlussglied (23), das dazu ausgebildet ist, die Einlassöffnung (5) zumindest teilweise zu verschließen,
- einem elastischen Rückstellorgan (26; 41; 42; 51), das dazu ausgebildet ist, das Verschlussglied (23) gegen die Einlassöffnung zu halten, um den Durchfluss von Flüssigkeit durch diese Einlassöffnung zu steuern,
- einem in der Kammer untergebrachten Rotor (4),
wobei das Ventil **dadurch gekennzeichnet ist, dass** der Rotor (4) eine Oberfläche aufweist, die einen Nocken (20) bildet, und dass das elastische Rückstellorgan (26; 41, 42; 51) sich an der genannten Oberfläche des Rotors abstützt und mit dieser Oberfläche einen beweglichen Kontakt bildet

2. Ventil nach Anspruch 1, **dadurch gekennzeichnet, dass** das elastische Rückstellorgan (26) an dem Körper des Ventils befestigt ist; insbesondere mit einem Ende.

3. Ventil nach einem der vorstehenden Ansprüche, **dadurch gekennzeichnet, dass** das elastische Rückstellorgan eine gekrümmte Blattfeder (26) aufweist.

4. Ventil nach Anspruch 3, **dadurch gekennzeichnet, dass** das elastische Rückstellorgan (26) eine gekrümmte Blattfeder ist, die mit einem Ende (27) an einer Seitenwand des Körpers des Ventils (2) befestigt ist und mit einem freien Ende (28) an dem Verschlussglied (23) anliegt.

5. Ventil nach Anspruch 3 oder 4, **dadurch gekennzeichnet, dass** die Blattfeder einen vorspringenden Abschnitt (30) aufweist, der an der den Nocken (20) bildenden Oberfläche anliegt.

6. Ventil nach Anspruch 5, **dadurch gekennzeichnet, dass** der vorspringende Abschnitt (30) durch Biegen der Blattfeder (26) gebildet ist.

7. Ventil nach Anspruch 3 oder 4, **dadurch gekennzeichnet, dass** das elastische Rückstellorgan ein an der Blattfeder (26') befestigtes Stützelement (36) aufweist, dass dazu ausgebildet ist, auf der den Nocken bildenden Oberfläche zu gleiten.

8. Ventil nach einem der Ansprüche 3 bis 7, **dadurch gekennzeichnet, dass** elastische Rückstellorgan eine einzige Blattfeder (26) aufweist.

9. Ventil nach einem der Ansprüche 3 bis 7, **dadurch gekennzeichnet, dass** das elastische Rückstellorgan wenigstens zwei zusammengebaute Blattfedern (26; 35) aufweist.

10. Ventil nach Anspruch 1, **dadurch gekennzeichnet, dass** das elastische Rückstellorgan (41) von dem Körper des Ventils unabhängig ist.

11. Ventil nach Anspruch 10, **dadurch gekennzeichnet, dass** das elastische Rückstellorgan eine Feder, insbesondere Schraubenfeder (41; 51) aufweist, die insbesondere an einem Ende mit einem Stützelement (42) verbunden ist, dass dazu eingerichtet ist, auf der den Nocken bildenden Oberfläche zu gleiten, wobei insbesondere das andere Ende der Feder an dem Verschlussglied anliegt.

12. Ventil nach Anspruch 11, **dadurch gekennzeichnet, dass** eine zusätzliche Feder (59) zwischen dem Stützelement (53) einerseits und dem Körper des Ventils oder einem an diesem Körper befestigten Einsatz andererseits angeordnet ist.

13. Ventil nach einem der vorstehenden Ansprüche, **dadurch gekennzeichnet, dass** die den Nocken bildende Oberfläche sich auf dem Umfang des Rotors erstreckt.

14. Ventil nach Anspruch 13, **dadurch gekennzeichnet, dass** die den Nocken bildende Oberfläche sich auf dem gesamten Umfang des Rotors erstreckt.

15. Ventil nach einem der vorstehenden Ansprüche, **dadurch gekennzeichnet, dass** die den Nocken bildende Oberfläche auf einem Winkelsektor von mehr als 180° einen zunehmenden Radius aufweist.

16. Ventil nach Anspruch 15, **dadurch gekennzeichnet, dass** die den Nocken bildende Oberfläche auf einem Winkelsektor, der im wesentlichen gleich 360° ist, einen zunehmenden Radius aufweist.

17. Ventil nach einem der vorstehenden Ansprüche, **dadurch gekennzeichnet, dass** die den Nocken bildende Oberfläche wenigstens eine Vertiefung (45), insbesondere mehrere um die Drehachse des Rotors verteilte Vertiefungen aufweist, in die ein Teil des elastischen Rückstellorgans eingreifen kann.

18. Ventil nach einem der vorstehenden Ansprüche, **dadurch gekennzeichnet, dass** das Verschlussglied eine Kugel (23) aufweist.

19. Ventil nach einem der vorstehenden Ansprüche, **dadurch gekennzeichnet, dass** der Rotor zwei Mikromagnete (12; 13) aufweist, die relativ zu dem Rotor (40) in einer im wesentlichen radialen Richtung linear beweglich sind und dazu eingerichtet sind, in einer vorbestimmten Winkelstellung mit Verriegelungsmitteln (18) des Rotors zusammenzuwirken.
